(19) **Europäisches Patentamt / European Patent Office / Office européen des brevets**

(11) **EP 4 631 527 A1**

(12) **EUROPEAN PATENT APPLICATION**
published in accordance with Art. 153(4) EPC

(43) Date of publication:
**15.10.2025   Bulletin 2025/42**

(21) Application number: **23901151.3**

(22) Date of filing: **08.12.2023**

(51) International Patent Classification (IPC):
**A61K 48/00** (2006.01)     **A61K 47/69** (2017.01)
**A61K 9/51** (2006.01)      **A61K 31/7088** (2006.01)
**A61P 31/12** (2006.01)

(52) Cooperative Patent Classification (CPC):
**A61K 9/51; A61K 31/7088; A61K 47/69;
A61K 48/00; A61P 31/12**

(86) International application number:
**PCT/KR2023/020203**

(87) International publication number:
**WO 2024/123134 (13.06.2024 Gazette 2024/24)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **09.12.2022   KR 20220171768**

(71) Applicants:
• **Seoul National University R&DB Foundation
  Seoul 08826 (KR)**
• **Nibec Co., Ltd.
  Chungcheongbuk-do 27816 (KR)**
• **Korea National Institute of Health
  Cheongju-si, Chungcheongbuk-do 28159 (KR)**

(72) Inventors:
• **PARK, Yoon Jeong
  Seoul 08291 (KR)**
• **CHUNG, Chong-Pyoung
  Seoul 05618 (KR)**
• **LEE, Jue-Yeon
  Gwacheon-si Gyeonggi-do 13831 (KR)**
• **YOON, Gookjin
  Incheon 21377 (KR)**
• **JO, Beom Soo
  Seoul 05817 (KR)**
• **LEE, Dong Woo
  Seoul 08213 (KR)**
• **KIM, Dokeun
  Cheongju-si Chungcheongbuk-do 28160 (KR)**
• **JEONG, Hye Sook
  Cheongju-si Chungcheongbuk-do 28160 (KR)**
• **SHIN, Eunkyung
  Cheongju-si Chungcheongbuk-do 28160 (KR)**

(74) Representative: **Mewburn Ellis LLP
Aurora Building
Counterslip
Bristol BS1 6BX (GB)**

Remarks:
The complete document including Reference
Table(s) and the Sequence Listing(s) can be
downloaded from the EPO website

(54) **NANOPARTICLE COMPRISING PEPTIDE-BASED CONJUGATE FOR DELIVERING MRNA INTO B CELL AND T CELL AND USES THEREOF**

(57)    The present invention relates to a peptide-based conjugate for mRNA delivery, which overcomes the limitations of existing lipid nanoparticles and is capable of safely and immediately responding to new variants of infectious diseases. mRNA binds to an RNA-binding peptide of the conjugate and undergoes self-assembly by an amphipathic polypeptide or polymer to thereby form a peptide and mRNA complex nanoparticle. The nanoparticle and a pharmaceutical composition comprising same were confirmed to efficiently increase intracellular delivery of mRNA and exhibit a vaccine effect by mRNA.

EP 4 631 527 A1

EP 4 631 527 A1

**FIG. 2**

mRNA

+

↓ Binding of mRNA to RNA-binding peptide

| RNA-binding peptide | Amphipathic polypeptide or polymer | Cell-penetrating peptide | Immune cell-recognizing peptide |

↓ self-assembly

NIPEP-TPP/mRNA complex

2

**Description**

**Technical Field**

The present invention relates to a peptide-based mRNA delivery carrier that may overcome the limitations of conventional lipid nanoparticles (LNPs) and ensure an immediate and safe response to new infectious diseases.

**Background Art**

Oligonucleotides have been developed as therapeutic agents for controlling genes that cause cancer and infectious diseases, especially in the form of siRNA and mRNA. Recently, as an mRNA vaccine encoding the spike protein of the SARS-CoV-2 virus in the form of mRNA, which induces an immune response upon injection thereof, was approved by the FDA, the application of mRNA has attracted attention. mRNA is nontoxic because artificially synthesized mRNA artificially synthesized *in vivo* is used, but it is rapidly degraded by nucleases *in vivo,* has a negative charge, and cannot target specific lesions. In addition, mRNA is effective only when it acts within the cytoplasm, but because of its negative charge and large size, it cannot penetrate the cell membrane by itself. Therefore, to overcome this limitation, a delivery carrier that is capable of stably delivering mRNA into cells is needed.

The most widely used carriers are lipid nanoparticles, and oligonucleotide carriers based on various cationic lipids are known (US 2006/0083780, US 2006/0240554, US 2008/0020058, US2009/0263407 and US 2009/0285881 and WO 2009/086558, WO 2009/127060, WO 2009/132131, WO2010/042877, WO 2010/054384, WO 2010/054401, WO 2010/054405, WO 2010/054406 and WO 2010/105209). Traditional cationic lipids such as CLinDMA and DLinDMA have been employed for oligonucleotide delivery to the liver, but are known to suffer from suboptimal delivery efficiency along with liver toxicity at higher doses.

Accordingly, the present inventors have prepared a peptide composed of a sequence for target-cell surface recognition, a cell-penetrating sequence, and a sequence capable of binding to infectious disease mRNA in order to produce a nanoparticle comprising a carrier peptide capable of safely and effectively delivering an infectious disease mRNA vaccine into cells, and have found that, when the nanoparticle comprising the carrier peptide is used, mRNA is effectively delivered into cells, and the loaded mRNA exhibits a vaccine effect, thereby completing the present invention.

The above information disclosed in this "Background Art" section is only for enhancement of understanding of the background of the present invention. Therefore, it may not include information that forms a conventional art that is already known in the art to which the present invention pertains.

**Summary of the Invention**

An object of the present invention is to provide a peptide-based conjugate capable of safely and effectively delivering mRNA into cells.

Another object of the present invention is to provide a nanoparticle for intracellular delivery of mRNA in which mRNA is bound to the peptide-based conjugate, and a composition for intracellular delivery of mRNA comprising the nanoparticle.

Still another object of the present invention is to provide a pharmaceutical composition for preventing or treating a viral infection comprising the nanoparticle.

Yet another object of the present invention is to provide a method for preventing or treating a viral infection comprising administering the nanoparticle.

Still yet another object of the present invention is to provide the use of the nanoparticle for preventing or treating a viral infection.

A further object of the present invention is to provide the use of the nanoparticle for the manufacture of a medicament for the prevention or treatment of a viral infection.

In order to achieve the above objects, the present invention provides a peptide-based conjugate for mRNA delivery having Structural Formula 1 below as a basic unit:

[Structural Formula 1]  A-B-D-E

wherein A is an RNA-binding peptide, B is an amphipathic polypeptide or an amphipathic polymer, D is a peptide having a cell-penetrating function, and E is a peptide that recognizes a B cell or T cell surface.

The present invention also provides a nanoparticle in which mRNA is bound to the peptide-based conjugate.

The present invention also provides a composition for delivery of mRNA into B cells or T cells comprising the nanoparticle in which mRNA is bound to the peptide-based conjugate.

The present invention also provides a pharmaceutical composition for preventing or treating a viral infection comprising the nanoparticle in which mRNA is bound to the peptide-based conjugate.

The present invention also provides a method for preventing or treating a viral infection comprising administering the nanoparticle.

The present invention also provides the use of the nanoparticle for preventing or treating a viral infection.

The present invention also provides the use of the nanoparticle for the manufacture of a medicament for the prevention or treatment of a viral infection.

**Brief Description of Drawings**

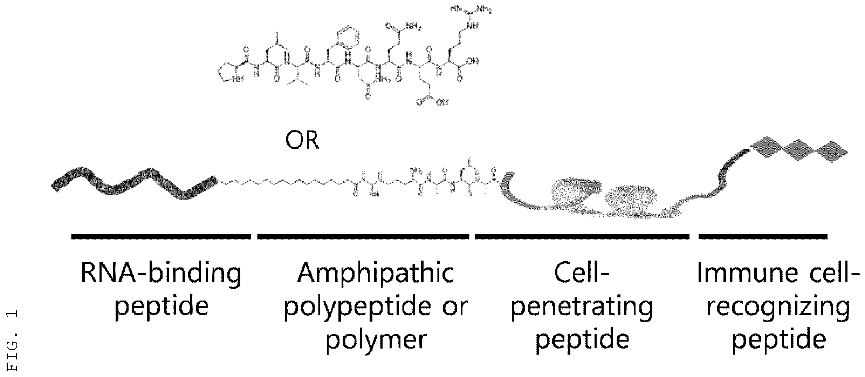

FIG. 1 is a schematic diagram showing the structure of a peptide-based conjugate.

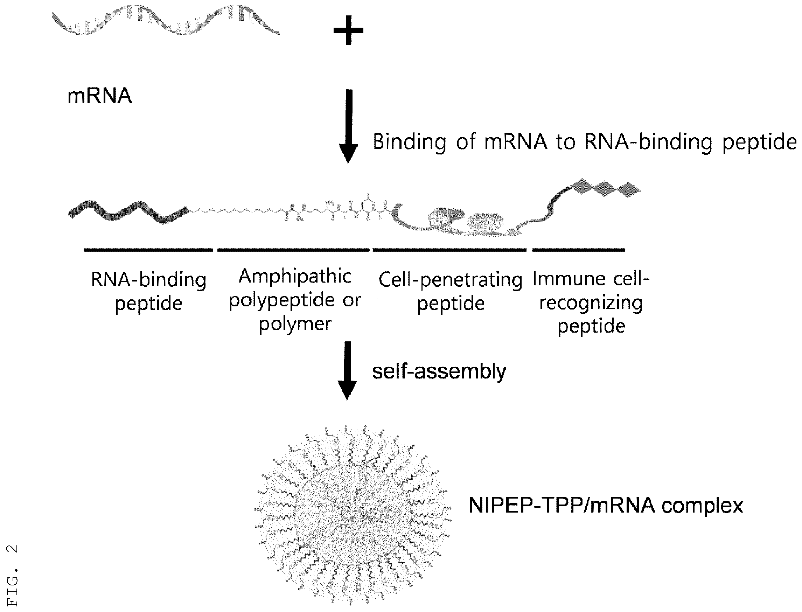

FIG. 2 is a schematic diagram showing the process of forming nanoparticles by binding of mRNA to the RNA-binding peptide of the peptide-based conjugate, followed by self-assembly.

FIG. 3A shows the results of electrophoresis of nanoparticles, composed of mRNA and the peptide-based conjugate, on agarose gel at each reaction ratio, and shows the results of electrophoresis after dissociation with 0.05% SDS, FIG. 3B shows the results of observing the formed nanoparticles using TEM, and FIG. 3C shows the results of measuring the size and distribution of the nanoparticles using an electrophoretic light scattering spectrophotometer.

FIG. 4 shows the results of observing the degree of intracellular penetration when the cells were treated with a conjugate of mRNA and lipofectamine or nanoparticles composed of mRNA and a peptide conjugate.

FIG. 5 shows the results of Western blot analysis performed to observe the expression level of intracellular protein by nanoparticles composed of mRNA and the peptide conjugate, and the results of Western blot analysis performed to confirm that active KRAS was reduced by the protein expressed from mRNA after treating cells with nanoparticles composed of mRNA and the peptide conjugate.

FIG. 6 shows the results of observing the binding of the B cell/T cell targeting peptide to B cells/T cells using confocal microscopy.

FIG. 7 shows the results of observing the binding of the B cell/T cell targeting peptide to B cells/T cells using flow cytometry.

FIG. 8 shows the results of TEM observation of nanoparticles produced with COVID-19 vaccine mRNA and the peptide carrier.

FIG. 9 shows the results of analysis of the anti-SARS-CoV-2 spike glycoprotein S1 antibody formed by the nanoparticles produced with COVID-19 vaccine mRNA and the peptide carrier.

FIG. 10 shows the results of analysis of the immune response formed by the nanoparticles produced with COVID-19 vaccine mRNA and the peptide carrier.

FIG. 11 shows the results of analysis of the antihuman ACE2 antibody formed by the nanoparticles produced with COVID-19 vaccine mRNA and the peptide carrier.

**Detailed Description and Preferred Embodiments of the Invention**

Unless otherwise defined, all technical and scientific terms used in the present specification have the same meanings as commonly understood by those skilled in the art to which the present disclosure pertains. In general, the nomenclature used in the present specification is well known and commonly used in the art.

In the present invention, nanoparticles comprising a peptide-based conjugate capable of safely and efficiently delivering mRNA into cells were produced. First, a peptide-based conjugate was produced using a peptide composed of a sequence for target cell surface recognition, a peptide having cell-penetrating function, a peptide facilitating endosomal escape, a peptide comprising a sequence capable of binding to an oligonucleotide, and an amphipathic polypeptide capable of protecting mRNA, and then nanoparticles were produced by binding mRNA to the conjugate. It has been found that, when the nanoparticles comprising the conjugate are used, the mRNA is effectively delivered into cells and that a vaccine effect against viral infection is exhibited by expression of the loaded mRNA.

Therefore, in one aspect, the present invention relates to a peptide-based conjugate for mRNA delivery having Structural Formula 1 below as a basic unit:

[Structural Formula 1] A-B-D-E

wherein A is an RNA-binding peptide,

B is an amphipathic polypeptide or an amphipathic polymer,

D is a peptide having a cell-penetrating function, and

E is a peptide that recognizes a B cell or T cell surface.

In the present invention, A may comprise a peptide sequence to which RNA binds. A may be a peptide represented by any one of the amino acid sequences of SEQ ID NO: 1 to SEQ ID NO: 15, without being limited thereto.

SEQ ID NO 1: LKKLLKLLKKLLKLAG
SEQ ID NO 2: IKKLIKIIKKLIKLAG
SEQ ID NO 3: LRRLLRLLRRLLRLAG
SEQ ID NO 4: LKKLLKLLOrnKLLDprLAG
SEQ ID NO 5: LRRLLRLLOrnRLLDprLAG
SEQ ID NO 6: LRKIIRLIOrnKLLDprLAG
SEQ ID NO 7: LKKLLKLLOrnKLLKLAG
SEQ ID NO 8: WKKLLKLLKKLLKLAG
SEQ ID NO 9: WRRLLRLLRRLLRLAG
SEQ ID NO 10: WRKLLRLLKKLLKLAG
SEQ ID NO 11: LKKLLDbuLLKKLLKWAG
SEQ ID NO 12: LRRLLDbuLLRRLLRWAG
SEQ ID NO 13: LKRLIDbuIIKKLIKWAG
SEQ ID NO 14: LKKLLKWLOrnKLLDprLAG
SEQ ID NO 15: LRRLLRWLOrnRLLDbuLAG
Orn = Ornithine, Dbu = 2,4-diaminobutyric acid, and Dpr = 2,3-diaminopropionic acid.

In the present invention, A may be used without limitation as long as it is a peptide to which RNA may bind. For example, A may be a nucleic acid-binding peptide disclosed in US 2020/0207834 A1, etc., without being thereto.

In the present invention, B may be an amphipathic polypeptide or an amphipathic polymer.

In the present invention, the term "amphipathic" means comprising hydrophilic and hydrophobic domains.

The amphipathic polypeptide refers to a polypeptide which contains polar amino acids and non-polar amino acids and in which the polar amino acids and nonpolar amino acids are partially densely packed together to form a polar region and a nonpolar region. The polar amino acids include cysteine, glutamine, threonine, tyrosine, serine, or asparagine, and the nonpolar amino acids include phenylalanine, tryptophan, methionine, proline, valine, isoleucine, leucine, glycine, or alanine, without being limited thereto.

In the present invention, the amphipathic polypeptide may be in a form in which polar amino acids and nonpolar amino acids are densely packed together in the amino acid sequence, or may be in a form in which polar amino acids and nonpolar amino acids are not densely packed together in the amino acid sequence, but when a peptide comprising the amino acids forms a three-dimensional structure, the polar amino acids and nonpolar amino acids may be densely packed together by the three-dimensional structure, without being limited thereto. For example, the amphipathic polypeptide may be a combination of a plurality of identical or non-identical polar amino acids and a plurality of identical or non-identical non-polar amino acids.

In one embodiment of the present invention, the amphipathic polypeptide may be a peptide represented by the amino acid sequence of SEQ ID NO: 16, without being limited thereto.

SEQ ID NO: 16: PLVFNQER

The amphipathic polymer is a polymer that is a combination of a hydrophobic polymer and a hydrophilic polymer, and is a polymer already known to those skilled in the art. The amphipathic polymer is a polymer that is a combination of hydrophilic and hydrophobic polymers, and may comprise any one of poloxamer, a poly(ethylene oxide) (PEO)-polylactic acid (PLA) copolymer, and a form in which the hydrophilic amino acid RALA is bound to the hydrophobic polymer polyetherimide (PEI), without being limited thereto. The amphipathic polymer may be a self-assembling amphipathic polymer selected according to the intended use. In one embodiment of the present invention, the amphipathic polymer may be in a form in which the hydrophilic amino acid RALA is bound to the hydrophobic polymer polyetherimide (PEI), without being limited thereto.

In the present invention, D may comprise a peptide sequence having a cell-penetrating function. The peptide having cell penetrating function is not particularly limited as long as it has the property of entering a cell by a cell internalization (endocytosis) mechanism, but is preferably selected from the group consisting of cell-penetrating peptides or variants thereof disclosed in Korean Patent No. 10-0951719.

In one embodiment of the present invention, D may be a peptide selected from the group consisting of 4 to 12 arginines, 4 to 12 lysines, and 2 cysteines, or a combination thereof, without being limited thereto, and peptides or peptide analogues other than the above-mentioned peptides may also be used as long as they can penetrate the cell membrane.

In the present invention, E may comprise a peptide sequence that recognizes a B cell or T cell surface.

The B cell or T cell refers to a cell targeted by mRNA that binds to the RNA-binding peptide and is to be delivered into the cell.

In the present invention, E is a sequence that binds to the B cell or T cell surface and may be newly discovered and applied through a phage display technique.

In one embodiment of the present invention, E may be a peptide represented by the amino acid sequence of SEQ ID NO: 17 that targets B cells and/or T cells, without being limited thereto.

SEQ ID NO: 17: HTHGAARVPDHR

In the present invention, the peptide-based conjugate may further comprise C, a peptide that facilitates endosomal escape, between B and D.

C may comprise a peptide sequence that facilitates endosomal escape, and is not particularly limited as long as it facilitates endosomal escape of the conjugate. Preferably, C may be selected from the group consisting of endosome-disrupting peptides or variants thereof disclosed in US 2020/0207834 A1.

In one embodiment of the present invention, C may be a peptide composed of 4 to 12 histidines, without being limited thereto.

In the present invention, A-B-D-E or A-B-C-D-E of Structural Formula 1 may be produced by a chemical synthesis technique or a recombinant expression technique.

In another aspect, the present invention relates to a nanoparticle in which mRNA is bound to the peptide-based conjugate.

In the present invention, the mRNA may encode a target protein, recombinant protein, or viral antigen whose expression is to be increased. The mRNA may be naive mRNA with a 3' cap and a 5' poly A tail, or may be bound to a sulfhydryl group of cysteine in the cell-penetrating and B/T cell-recognizing peptide using a chemical cross-linking agent after binding the sulfhydryl group or amine group to the 5' end.

In one example of the present invention, mRNA against mutant KRAS was used to inhibit the expression of mutant KRAS. The mRNA sequence encoding a protein that degrades KRAS may use mRNA having the nucleotide sequences of SEQ ID NO: 18 and SEQ ID NO: 19, without being limited thereto.

<u>SEQ ID NO: 18: mRNA sequence 1 encoding protein that degrades KRAS</u>

5' cap-5'UTR-

AUGGAAGUCCAAUUGUUGGAAUCUGGGGGUGGGCUUGUCCAACCUGGUGGUUCA

CUCCGUCUGAGCUGCGCCGCUUCCGGAUUCACUUUCUCCACCUUUUCUAUGAAUUGGGU

UCGCCAGGCCCCAGGCAAGGGACUUGAGUGGGUUAGCUAUAUCAGCAGGACCAGUAAAA

CCAUCUACUACGCCGACAGCGUUAAGGGAAGGUUCACUAUAUCCCGGGACAAUUCUAAG

AAUACUUUGUACUUGCAAAUGAACUCUUUGCGGGCAGAAGAUACUGCUGUCUACUAUUG

UGCACGAGGUAGGUUUUUUGAUUAUUGGGGUCAGGGGACCCUCGUAACCGUUUCCAGUG

GGGGCAGCGAGGGUAAGAGCUCCGGUUCAGGGUCAGAAUCUAAAAGCACUGGUGGCUCC

GAUAUACAAAUGACCCAGAGUCCAAGCUCUCUUUCAGCCAGCGUGGGAGACAGGGUGAC

CAUCACCUGUCGAGCAUCUCAAUCCAUUUCAUCAUACCUCAAUUGGUAUCAACAGAAGC

CUGGGGAAGCACCAAAGUUGCUUAUUUAUUCUGCCAGCGUACUGCAAAGUGGCGUACCU

UCCCGCUUCUCCGGGUCUGGGAGUGGCACUGAUUUUACCCUCACAAUUAGCAGCCUGCA

ACCCGAGGACUUCGCUACAUACUACUGCCAACAAAGUGUAAUGAUACCCAUGACAUUCG

GGCAAGGCACAAAAGUCGAGAUAAAGGGAAGUGGAGGAGGUGGCUCUAUGCCCCGCCGC

GCUGAGAACUGGGAUGAGGCCGAAGUUGGUGCUGAAGAGGCCGGAGUUGAAGAAUAUGG

GCCAGAAGAAGAUGGCGGUGAGGAAAGCGGGGCUGAAGAGAGCGGUCCAGAGGAGUCUG

GUCCAGAGGAACUUGGAGCUGAGGAGGAGAUGGAGGCCGGACGUCCACGUCCUGUCCUG

CGAUCAGUUAACUCAAGGGAGCCCUCACAGGUUAUCUUUUGUAAUCGAAGCCCUCGCGU

GGUUUUGCCCGUCUGGCUCAACUUCGAUGGUGAACCCCAACCAUACCCAACCCUCCCCC

CUGGUACCGGGCGGAGGAUUCAUUCCUAUCGGGGACACCUUUGGCUCUUCCGAGACGCU

GGGACUCACGAUGGCCUGUUGGUCAAUCAGACCGAACUGUUUGUGCCCAGCCUGAACGU

AGACGGGCAGCCAAUUUUCGCAAACAUAACACUGCCCGUAUAUACAUUGAAAGAAAGGU

GUCUUCAGGUGGUACGCAGUCUGGUUAAACCAGAAAACUAUAGGCGUCUGGAUAUCGUG

CGCAGCCUCUAUGAGGACCUUGAAGACCACCCUAACGUGCAAAAGGACCUCGAGCGGCU

CACUCAGGAGCGCAUAGCUCAUCAGCGCAUGGGAGAUGAAAACCUUUACUUUCAGGGUG

GGAGCGGCGGCAGCGGCGGGAGUCAUCAUCACCAUCAUCAUCACCACUGA-3' UTR-

3' poly A tail

SEQ ID NO: 19: mRNA sequence 2 encoding protein that degrades KRAS

5' cap-5'UTR-

AUGGAAGUGCAACUUCUCGAAUCUGGAGGGGGGUUGGUACAACCCGGCGGCAGC

UUGCGCUUGUCCUGUGCUGCCAGUGGGUUCACAUUUUCUACAUUCUCUAUGAACUGGGU

UAGACAAGCCCCUGGCAAAGGGCUGGAAUGGGUGAGCUAUAUAAGUCGCACCUCCAAAA

CUAUUUACUAUGCAGAUAGUGUCAAGGGACGAUUUACUAUCAGCAGAGAUAACUCUAAG

AACACUCUGUAUCUCCAGAUGAAUUCCCUUCGAGCCGAAGACACCGCAGUAUAUUACUG

UGCUAGAGGGCGCUUCUUCGACUACUGGGGGCAAGGCACUCUGGUAACUGUCAGUAGCG

GGGGUUCUGAGGGCAAAAGUUCAGGAUCUGGGUCCGAGUCUAAGUCAACUGGCGGGAGC

GACAUACAAAUGACUCAGAGCCCCAGUAGCCUGAGCGCCUCCGUUGGUGACAGAGUCAC

UAUUACAUGUCGGGCAUCUCAAUCUAUUUCCUCUUAUCUUAACUGGUAUCAGCAAAAGC

CAGGCGAAGCCCCCAAACUCCUGAUAUACUCCGCUAGUGUUCUUCAGAGUGGCGUUCCU

```
AGUCGCUUCAGCGGAUCCGGAAGUGGCACUGAUUUUACACUUACAAUUAGUUCCUUGCA

GCCUGAAGAUUUGCCACAUACUACUGUCAGCAAUCAGUCAUGAUUCCCAUGACCUUUG

GGCAAGGGACCAAAGUUGAGAUCAAAGGCUCCGGAGGAGGAGGGUCUAUGGAAAACAGA

UGGCAAGUUAUGAUCGUUUGGCAAGUAGAUCGUAUGCGAAUACGCACAUGGAAAUCACU

GGUAAAGCACCACAUGUAUGUGUCAGGCAAAGCACGAGGAUGGUUUUAUCGUCAUCACU

AUGAGUCACCACAUCCCCGCAUCAGCUCUGAGGUGCACAUACCACUCGGUGAUGCUCGU

CUGGUCAUAACUACCUACUGGGGCCUCCACACUGGUGAAAGAGAUUGGCACCUCGGCCA

AGGAGUAAGUAUAGAAUGGCGCAAAAAGCGUUAUUCAACCCAAGUGGAUCCUGAGUUGG

CAGAUCAGUUGAUCCACUUGUAUUACUUUGACUGCUUCAGCGAUAGUGCUAUUCGGAAG

GCCCUCCUCGGGCACAUUGUAAGCCCACGGUGCGAGUAUCAAGCUGGUCAUAACAAGGU

UGGUAGCCUCCAGUACUUGGCUUUGGCUGCACUUAUAACACCCAAAAAGAUAAAACCUC

CACUCCCCUCAGUGACUAAACUCACUGAGGAUCGUUGGAACAAACCCCAGAAAACCAAG

GGGCACAGGGGUUCACACACCAUGAAUGGCCACGAGAAUUUGUAUUUCCAAGGGGGAUC

CGGGGGCAGUGGAGGGUCUCACCAUCAUCACCACCACCACCAUUGA-3'    UTR-3'

poly A tail
```

In the present invention, after producing Structural Formula 1, mRNA may be bound to Structural Formula 1, specifically, A in Structural Formula 1, and allowed to stand for a predetermined period of time, so that nanoparticles may be formed by self-assembly.

The nanoparticle may have a size of 10 to 200 nm, without being limited thereto.

In the present invention, the molecular weight ratio between the mRNA and the conjugate may be 1:1 to 1:100.

In the present invention, the mRNA may be used as an mRNA vaccine, without being limited thereto.

In still another aspect, the present invention relates to a composition for delivering mRNA into B cells or T cells comprising the nanoparticle in which mRNA is bound to the peptide-based conjugate.

In yet another aspect, the present invention relates to a pharmaceutical composition for preventing or treating a viral infection comprising the nanoparticle in which mRNA is bound to the peptide-based conjugate.

The viral infection may be a viral infection including a cold, flu (influenza), infectious mononucleosis, cytomegalovirus infection, measles, polio, yellow fever, dengue fever, hepatitis B, hepatitis C, AIDS, or COVID-19, without being limited thereto.

In the present invention, the term "prevention" means any action that suppresses or delays a disease by administering the composition, and the term "treatment" means any action that ameliorates or completely cures the symptoms of a disease by administering the composition.

According to the present invention, the pharmaceutical composition may be prepared in any one dosage form selected from the group consisting of injections, preparations for oral administration, patches, solutions, capsules, granules, tablets, powders, sprays, ointments, gels, mucosal preparations, and suppositories, without being limited thereto. These dosage forms may be prepared by conventional methods used for formulation in the art or by a method disclosed in Remington's Pharmaceutical Science (latest edition), Mack Publishing Company, Easton PA, and the pharmaceutical composition may be prepared in various dosage forms depending on each disease or ingredient. However, the description is illustrative and dosage forms to which the present invention is applicable are not limited to those described above.

In the present invention, the pharmaceutical composition may further comprise an acceptable excipient, and the excipient may be, for example, a carrier. A pharmaceutically acceptable carrier may be saline, sterile water, Ringer's solution, buffered saline, dextrose solution, maltodextrin solution, glycerol, ethanol, or a mixture of one or more of these components, and, if necessary, the pharmaceutical composition may further comprise other conventional additives such as antioxidants, buffers, or bacteriostatic agents. In addition, a diluent, a dispersant, a surfactant, a binder, and a lubricant may be additionally added to prepare a pill, capsule, granule, or tablet or to prepare an injectable dosage form such as an aqueous solution, suspension, or emulsion. However, the above description is illustrative and the adjuvant or carrier usable in the present invention is not limited to those described above.

In still yet another aspect, the present invention relates to a method for preventing or treating a viral infection comprising administering the nanoparticle.

In a further aspect, the present invention relates to the use of the nanoparticle for the prevention or treatment of a viral infection.

In another further aspect, the present invention relates to the use of the nanoparticle for the manufacture of a medicament for the prevention or treatment of a viral infection.

In the present invention, the method and use comprise the nanoparticle according to the present invention, and relate to the pharmaceutical composition comprising the nanoparticle, and the description of contents overlapping with the above-described nanoparticle and pharmaceutical composition are omitted

Hereinafter, the present invention will be described in more detail by way of examples. These examples are only intended to illustrate the present invention, and it will be apparent to those skilled in the art that the scope of the present invention is not to be construed as being limited by these examples.

## Example 1: Synthesis of Carrier Peptide

Amino acids and reagents required for synthesis were purchased from GL Biochem and Sigma-Aldrich. A peptide was synthesized from the C-terminus by the Fmoc solid-phase chemical synthesis method using a peptide synthesizer. That is, a peptide was synthesized using a rink resin (0.075 mmol/g, 100 to 200 mesh, 1% DVB crosslinking) to which Fmoc-(9-fluorenylmethoxycarbonyl) was bound as a blocking group. 50 mg of the rink resin was placed in the peptide synthesizer, the resin was swelled with DMF, and then a 20% piperidine/DMF solution was used to remove the Fmoc-group. According to the sequence from the C-terminus, a 0.5M amino acid solution (solvent: dimethylformamide, DMF), 1.0M DIPEA (solvent: dimethylformamide & n-methylpyrrolidone, DMF&NMP), and 0.5M HBTU (solvent: dimethylformamide, DMF) were added to the resin in amounts of 5, 10, and 5 equivalents, respectively, and allowed to react under a nitrogen atmosphere for 1 to 2 hours. After completion of each of the deprotection and coupling steps, the resin was washed twice with DMF and isopropanol. Even after coupling the last amino acid, deprotection was performed to remove the Fmoc-group. After completion of the reaction, the resin was washed with DMF and MeOH and dried in a vacuum oven. A trifluoroacetic acid (TFA) cleavage cocktail was added to the resin in an amount of 20 ml per g of the resin, followed by shaking for 3 hours, and then a cocktail containing the resin and peptide dissolved therein was separated by filtration. Cold ether was added in excess directly to the filtered TFA cocktail solution containing the peptide dissolved therein to crystallize the peptide into a solid phase, which was then separated by centrifugation. At this time, the TFA cocktail was completely removed by washing several times with ether and a centrifugation process. The peptide thus obtained was dissolved in distilled water and lyophilized. After completion of lyophilization, the peptide was separated and purified by high-performance liquid chromatography (Shimadzu, Japan). Analysis was performed using a $C_{18}$ column with a diameter of 4.6 mm by flowing 0.1% TFA/$H_2O$ and 0.092% TFA/acetonitrile with a change of 0 to 60% at a flow rate of 1 ml/min for 30 minutes. At this time, the wavelength of the ultraviolet detector was 220 nm. Purification was performed using a column with a diameter of 2.2 cm at a flow rate of 20 ml/min under the same solvent and detection wavelength conditions. The molecular weight of the purified peptide was determined by mass spectrometry.

## Example 2: Production of Nanoparticles by Binding of mRNA to Carrier Peptide

Nanoparticles were produced by binding mRNA of a gene encoding a His tag protein or an enhanced green fluorescent protein (eGFP) protein to the carrier peptide produced in Example 1.

5 mg of the carrier peptide was mixed with and dissolved in 1 ml of nuclease-free water (Invitrogen, AM9938). 1 mg of mRNA was dissolved in 1 ml of nuclease-free water (Invitrogen, AM9938). The carrier peptide solution and the mRNA solution were slowly mixed in small amounts at nitrogen/phosphate (N/P) ratios of 1:1, 2:1, 3:1, 4:1, 5:1, 10:1, and 20:1.

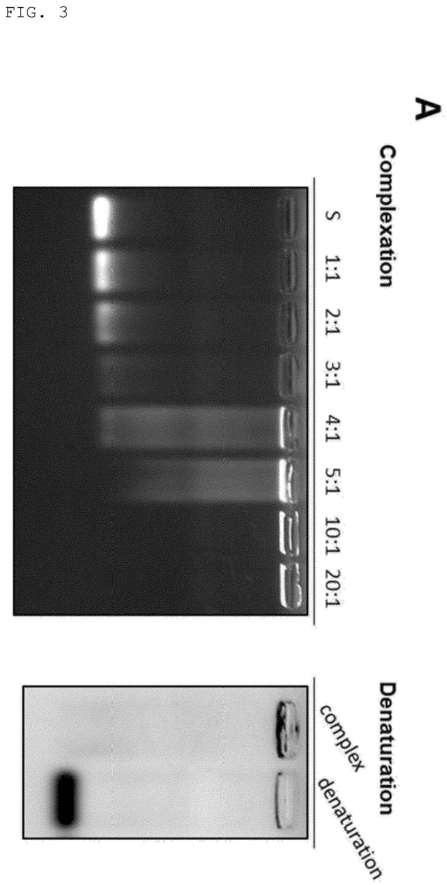

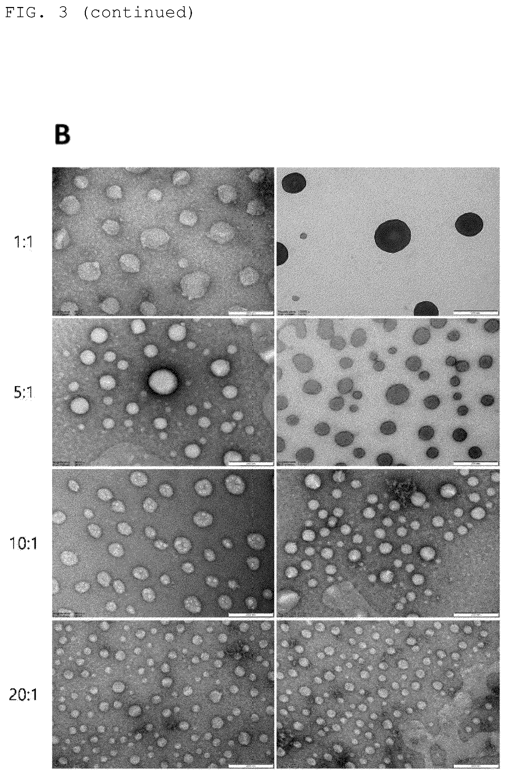

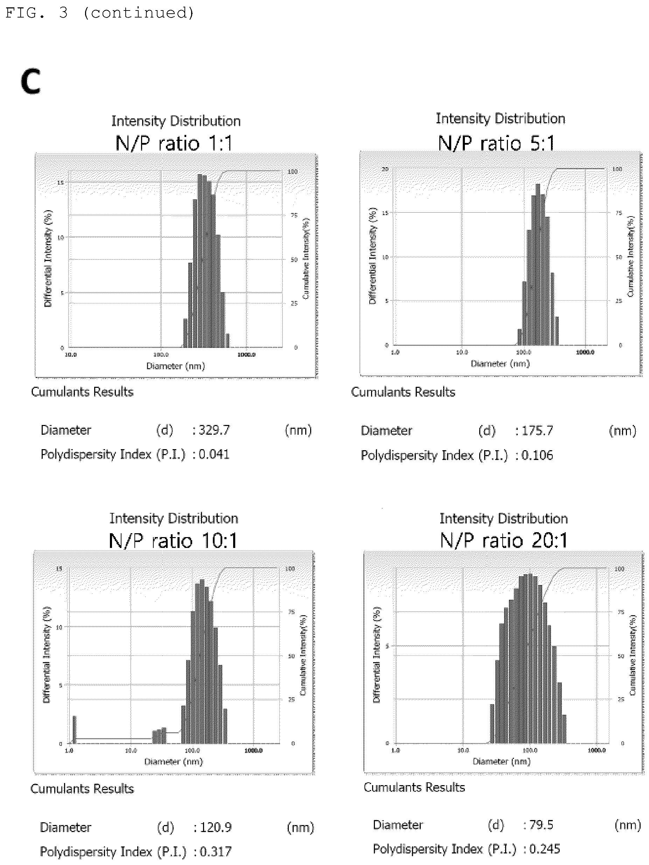

The formed nanoparticles were identified by a gel retardation test. mRNA and the carrier peptide were loaded on 2% (w/v) agarose gel in an amount of mRNA corresponding to 0.1 μg at ratios of 1:1, 2:1, 3:1, 4:1, 5:1, 10:1, and 20:1, followed by electrophoresis. It was observed that single mRNA migrated downward with a positive (+) charge (FIG. 3), but it could be seen that, when nanoparticles were formed, single mRNA did not migrate downward at a ratio of 10:1 or higher. This means that the particle surface was neutralized by the carrier peptide. In addition, it could be confirmed that, when the formed nanoparticles were dissociated with 0.05% SDS, the mRNA came down to the gel again (FIG. 3). In addition, nanoparticles comprising the conjugate of mRNA and the carrier peptide were observed using a transmission electron microscope (TEM, JEM-1230, JEOL). The particle size and zeta potential were measured using an electrophoretic light scattering spectrophotometer (ELS Z-1000, Otsuka Portal, Japan) (FIG. 3). It was confirmed that the produced nanoparticles had a diameter of 100 to 150 nm.

## Example 3: Confirmation of Delivery of Nanoparticles into Cells and Protein Expression

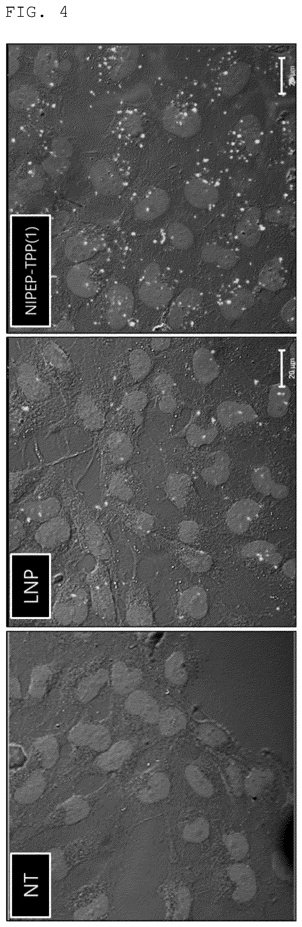

The nanoparticles produced in Example 2 were added to a culture of Raw264.7 cells, and it was confirmed that protein was expressed in the cells. Nanoparticles with the carrier peptide were formed using mRNA expressing eGFP, and it was observed by confocal microscopy that the nanoparticles were delivered into the cells and that the eGFP protein was expressed in the cells (FIG. 4).

To test the cell penetration ability of nanoparticles comprising the conjugate of mRNA and the carrier peptide, the nanoparticles were produced in the same manner as in Example 2. Raw264.7 (murine macrophage cells, TIB-71, ATCC) were seeded at a density $1 \times 10^4$, and after 24 hours, the culture medium was treated with 20 $\mu$g (amount of mRNA) of the conjugate of mRNA and the carrier peptide for 24 hours. Then, the cells were fixed with a 10% neutral formalin solution and measured using a confocal scanning microscope

As a result, a significantly increased amount of the nanoparticles was observed in the cytoplasm of the Raw264.7 cells (FIG. 4). Therefore, it could be confirmed that the nanoparticles comprising the conjugate of mRNA and the carrier peptide were capable of effectively penetrating the cells.

## Example 4: Identification of Expression of KARS Degradation Protein and Reduction of KRAS by mRNA Delivered into Cells

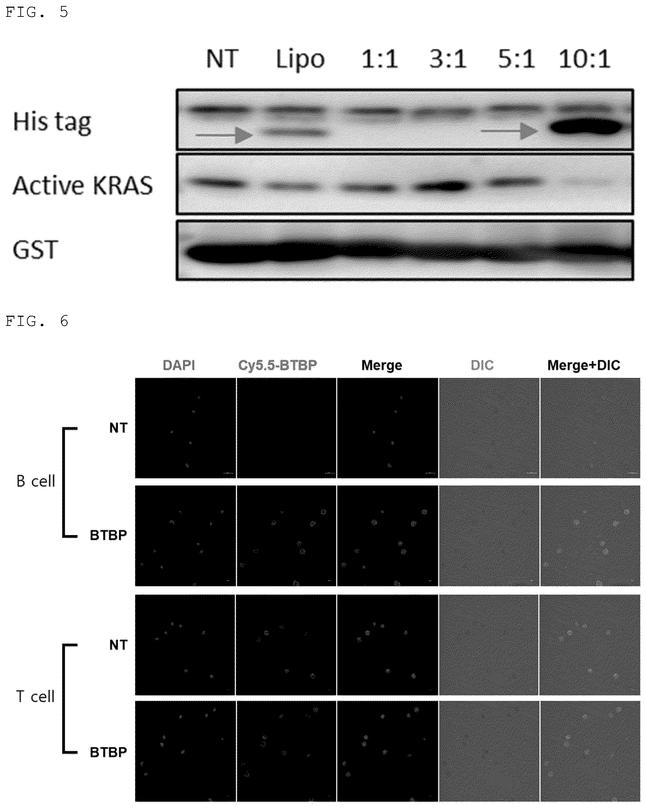

In addition, the amount of protein expressed in cells the nanoparticles comprising mRNA and the carrier peptide conjugate was analyzed by Western blot analysis (FIG. 5).

H358 cells were seeded in a 6-well plate at a density of 60%. After 16 hours, the cells were starved with serum free RPMI-1640 overnight. The cells were treated with a medium containing 20 $\mu$g (amount of mRNA) of the nanoparticles comprising the conjugate of mRNA and the carrier peptide for 20 hours. 20 $\mu$g mRNA mixed with lipofectamine was used as a control. Protein lysis was performed using RIPA lysis buffer (25 mM Tris·HCl pH 7.6, 150 mM NaCl, 1% NP-40, 1% sodium deoxycholate, 0.1% SDS) containing protease and phosphatase inhibitors. Protein quantification was performed by BCA protein assay (23227, Thermo Scientific), and the expression level of His tag protein was analyzed by Western blot analysis. For Western blot analysis, equal amounts of samples were loaded onto a 12% SDS PAGE gel along with a size marker, electrophoresed for about 2 hours, and then transferred to a nitrocellulose membrane. The transferred membrane was blocked with 5% skim milk for 1 hour, and incubated with a primary antibody (His tag, Abcam, ab18184) at a ratio of 1:1,000 overnight. Thereafter, the membrane was washed with TBST containing 0.1% Tween-20, and incubated with an HRP-conjugated secondary antibody (anti-mouse, A120-101P, BETHYL Lab.) for 1 hour, and then chemiluminescence was detected with an ECL substrate.

As a result, as shown in FIG. 5, it was confirmed that, in the case of the nanoparticles comprising the carrier peptide-mRNA conjugate according to the present invention, the amount of the protein expressed by the mRNA in the cells was higher at a higher N/P ratio than when lipid nanoparticles such as lipofectamine were used.

In addition, to confirm the expression of GTP-bound KRAS (active KRAS), the cell lysate was incubated with RBD-RAF-GST (RAS binding domain RAF GST) and beads for 1 hour. The beads were washed three times with lysis buffer, and the protein was dissociated from the beads using 2x loading buffer. For Western blot analysis, equal amounts of samples were loaded onto a 12% SDS PAGE gel along with a size marker, electrophoresed for about 2 hours, and then transferred to a nitrocellulose membrane. The transferred membrane was blocked with 5% skim milk for 1 hour, and incubated with primary antibodies (GST (Abcam, ab19256), KRAS (Santa Cruz, sc-30)) at a ratio of 1:1,000. Thereafter, the membrane was washed with TBST containing 0.1% Tween-20, and incubated with HRP-conjugated secondary antibodies (anti-rabbit, A90-116P, BETHYL Lab.; anti-mouse, A120-101P, BETHYL Lab.) for 1 hour, and then chemiluminescence was detected with an ECL substrate.

As a result, as shown in FIG. 5, it was confirmed that the expression of active KRAS was significantly reduced in the nanoparticles in which the protein was expressed.

## Example 5: Confirmation of Ability of B Cell/T Cell Targeting Peptide to Target B Cells/T Cells

A B cell/T cell targeting peptide (BTBP, SEQ ID NO: 17) was labeled with Cy5.5, and B cells (primary CD19+ B cells, PCS-800-018, ATCC) and T cells (primary CD8+ cytotoxic T cells, PCS-800-017, ATCC) were treated with 100 $\mu$M of the peptide for 1 hour. Then, the cells were fixed with 4% neutral formalin solution and measured using a confocal scanning microscope (LSM980 with Airyscan2, Carl Zeiss). As a result, it was observed that the fluorescence intensity significantly increased in the cell membranes of the B cells and the T cells (FIG. 6). Therefore, it was confirmed that BTBP effectively targeted both the B cells and the T cells.

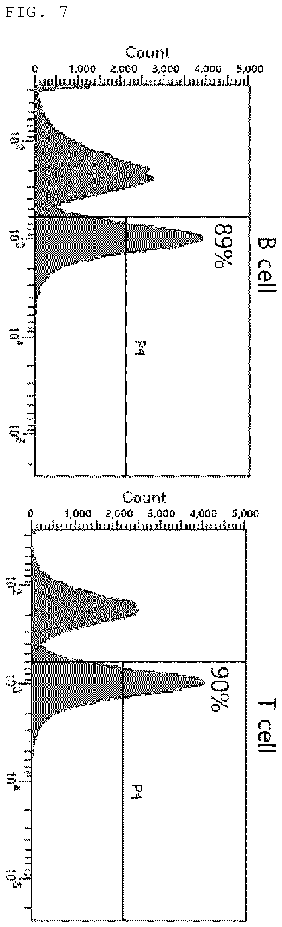

In addition, the cells were washed three times with DPBS containing 5% FBS, and cells bound with Cy5.5-labeled BTBP were analyzed using flow cytometry (FIG. 7). As a result, it could be confirmed that the peptide of SEQ ID NO: 17 bound to B cells and T cells.

**Example 6: Nanoparticle Production**

COVID-19 vaccine mRNA and the RNA-binding peptide (SEQ ID NO: 1 or 3) were set at a volume ratio of 1:1, and they were reacted at 1.3 ml/min after connecting the cartridges to the NanoAssemblr Ignite (Precision Nanosystems). After the first complexation, incubation was performed at room temperature for 30 minutes. Thereafter, 2 ml of the first complexation sample and 1 ml of the amphipathic polypeptide (SEQ ID NO: 16) were reacted at a volume ratio of 2:1, a final volume of 3 ml, and 1.3 ml/min after connecting the cartridges to the NanoAssemblr Ignite. After the second complexation, incubation was performed at room temperature for 30 minutes. Next, 3 ml of the sample obtained by the second complexation and 1 ml of the B cell/T cell binding peptide (SEQ ID NO: 17) were reacted at a volume ratio of 3:1, a final volume of 4 ml, and 1.3 ml/min after connecting the cartridges to the NanoAssemblr Ignite. Finally, the final sample was filtered using a cellulose acetate filter (0.2 $\mu$m) and concentrated with nitrogen gas to a volume of 200 $\mu$l. The following samples with two weight ratios between the nanoparticle components mRNA:RNA binding peptide:amphipathic polypeptide:B cell/T cell binding peptide were prepared.

```
Sample 1 = 1:2:2:5

Sample 2 = 1:3:3:5
```

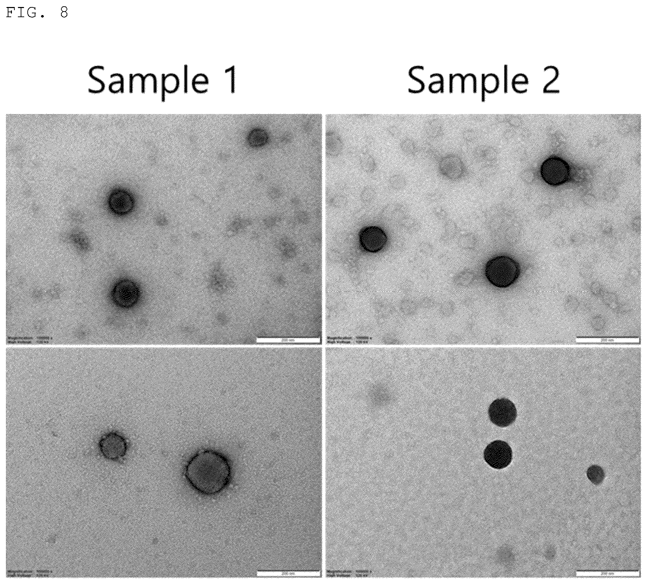

LNPs used as a control were prepared by mixing organic and aqueous phases so that they contained mRNA at a certain weight ratio. The organic phase was prepared by mixing ionized lipid (SM-102, Moderna), phospholipid (DSPC; Avanti, 816-94-4), cholesterol (Cholesterol; Avanti 700100P), and PEG lipid (DMG-PEG; Avanti 880151P) at a ratio of ionized lipid:phospholipid:cholesterol:PEG lipid = 50 (6.25 mM):10 (1.25 mM):38.5 (4.8125 mM):1.5 (1.875 mM) in ethanol (Sigma E7023). The aqueous phase was prepared by mixing mRNA with citrate (Sigma 854) and DPBS (Cytiva SH30028.02). The organic phase and the aqueous phase were adjusted to a weight ratio of 1:3 and mixed at a flow rate of 12 ml/min to produce LNPs. The produced LNPs were dialyzed against PBS for 16 hours using a dialysis cassette (Merk, UFC901024) to remove ethanol and adjust the pH of the LNPs to *in vivo* pH. The size and shape of the produced nanoparticles were observed using TEM (JEM-1400, JEOL) (FIG. 8). As a result, both sample 1 and sample 2 showed a size distribution of 129 to 155 nm, and both had a spherical shape.

**Example 7: Evaluation of Formation of Anti-SARS-CoV-2 Spike Glycoprotein S1 Antibody Using ELISA**

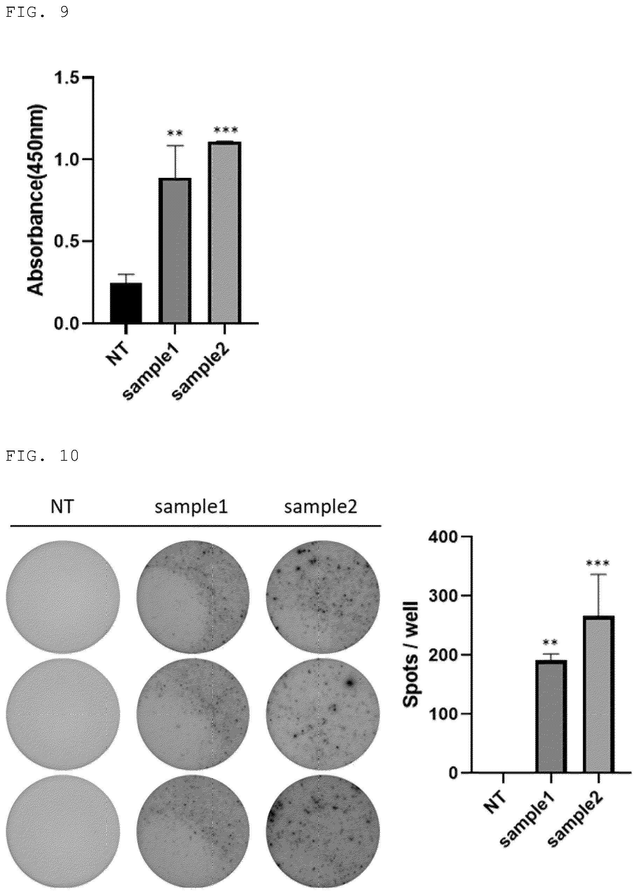

The nanoparticles produced in Example 6 were injected into the thigh muscle of C57BL/6 mice, and after 2 weeks, the mice were sacrificed. The collected blood was centrifuged to separate the serum. Recombinant human coronavirus SARS-CoV-2 spike glycoprotein S1 (ab275927) in ELISA coating buffer (100 ng/50 $\mu$l) was added to each well, and the wells were coated at 4°C for 18 hours. The coating buffer on the plate was removed and the plate was washed twice with washing buffer (PBST, PBS+0.05% Tween20). 100 $\mu$l of PBS+1% BSA buffer was added to each well which was then blocked by incubation at 37°C for 2 hours. After each well was washed twice with washing buffer, the separated serum was diluted serially diluted 2-fold from 1:40 in PBS + 1% BSA buffer, and added to each well at 100 $\mu$l/well, and each well was incubated in an incubator at 37°C for 1 hour. After each well was washed three times with washing buffer, horseradish peroxidase (HRP)-labeled secondary antibody was diluted at 1:5,000 and added to each well at 50 $\mu$l/well, and each well was incubated at 37°C for 1 hour. After each well was washed five times with washing buffer, TMB solution was added to each well at 50 $\mu$l/well, and each well was reacted at RT for 10 minutes. Then, stop solution (2 M $H_2SO_4$) was added to each well at 50 $\mu$l/well to stop the reaction. The absorbance at a wavelength of 450 nm was measured using a microplate reader (FIG. 9). As a result, it was confirmed that the experimental groups injected with sample 1 and sample 2 exhibited high absorbance, indicating that an antibody against SARS-CoV-2 spike glycoprotein S1 was formed. Therefore, it was proven that the injected nanoparticles delivered COVID-19 vaccine mRNA into the body and effectively expressed SARS-CoV-2 spike glycoprotein S1 to induce an immune response.

**Example 8: Evaluation of Immune Response Using ELISPOT**

The nanoparticles produced in Example 6 were injected into the thigh muscle of C57BL/6 mice, and the mice were sacrificed 2 weeks later. The mouse spleen was extracted, ground using a 40-$\mu$m pore strainer in 5 ml of 1x HBSS medium, transferred into a 50-ml conical tube, and centrifuged at 2,000 rpm for 5 minutes. After removing the medium, 5 ml of ACK buffer was added to the cells which were then incubated at room temperature for 5 minutes to remove red blood cell, thus obtaining splenocytes. The splenocytes obtained from the spleen of the mice immunized with the candidate substance were dispensed onto a microplate coated with IFN-$\gamma$ antibody. Then, the cell culture medium was added to a negative control group, a cell stimulation cocktail to a positive control group, and an SARS-CoV-2 spike peptide (Genscript RP30020 SARS-CoV-2 spike glycoprotein-crude, 2 vials (25 $\mu$g/peptide)) to an experimental group, followed by incubation in a $CO_2$ incubator at 37°C for 18 hours. Biotinylated IFN-$\gamma$ monoclonal antibody was added to each well which was then incubated at room temperature for 1 hour. Next, streptavidin-alkaline phosphatase was added to each well which was then incubated at room temperature for 1 hour. 5-bromo-4-chloro-3-indolyl-phosphate/nitro blue tetrazolium (BCIP/NBT) solution was added to each well which was then incubated at room temperature for 10 minutes. After washing, the color spots were calculated and analyzed (FIG. 10). As a result, it was confirmed that the SARS-CoV-2 spike peptide induced an immune response in the spleen cells isolated from the mice injected with each of sample 1 and sample 2. Therefore, it was proven that the injected nanoparticles delivered COVID-19 vaccine mRNA into the body and effectively expressed SARS-CoV-2 spike glycoprotein S1 to induce an immune response.

**Example 9: Evaluation of Formation of Antibody against Human ACE2 using hACE2 Immunoassay**

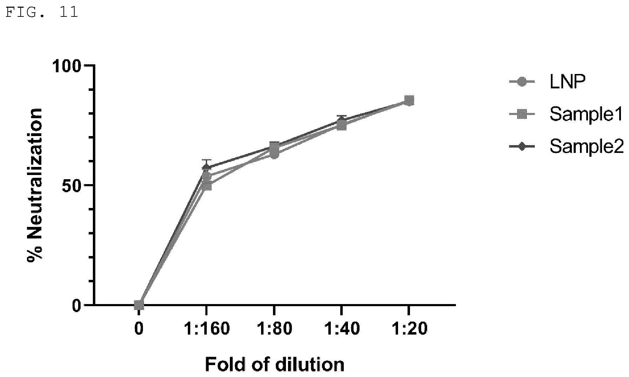

The nanoparticles produced in Example 6 were injected into the thigh muscles of C57BL/6 mice, and the mice were sacrificed 2 weeks later. The collected blood was centrifuged to separate the serum. After serum separation, the serum was heat-inactivated at 56°C for 30 minutes. This experiment was conducted using the Lumit™ SARS-CoV-2 Spike RBD: hACE2 Immunoassay kit from Promega. First, a 10X sample mix was prepared by diluting the serum in 1X immunoassay reaction buffer, and 5 $\mu$l of the sample mix was dispensed into each well of a 96-well white plate. Then, 10 $\mu$l of rFc-RBD reagent of III.B.2 was added to each well. Next, 10 $\mu$l of mFc-ACE2 reagent of III.B.2 was added to each well. Then, 25 $\mu$l of Lumit™ antibody mix reagent of III.B.3 was added to all the wells, and the reagents were mixed for 2 minutes at room temperature on a plate shaker and incubated for 60 minutes. Finally, 12.5 $\mu$l of Lumit™ detection reagent was added to each well, followed by incubation at room temperature for 30 minutes. In addition, luminescence was measured using a luminescence microplate reader (FIG. 11). As a result, it was confirmed that an antibody against human ACE2 was formed in the serum of the mice injected with each of sample 1 and sample 2. Therefore, it was proven that the injected nanoparticles delivered COVID-19 vaccine mRNA into the body and effectively expressed SARS-CoV-2 spike glycoprotein S1 to generate an immune response.

**Industrial Applicability**

The nanoparticles comprising the mRNA-bound peptide-based conjugate according to the present invention can effectively deliver mRNA into cells and may be used as a vaccine for preventing infections by mRNA. The nanoparticle according to the present invention may enhance the vaccine effect of mRNA by improving expression efficiency, safety, etc. compared to existing lipid nanoparticles.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

**Sequence Listing Free Text**

An electronic file is attached.

**Claims**

**1.** A peptide-based conjugate having Structural Formula 1 below as a basic unit:

[Structural Formula 1]        A-B-D-E

wherein A is an RNA-binding peptide,

B is an amphipathic polypeptide or an amphipathic polymer,

D is a peptide having a cell-penetrating function, and

E is a peptide that recognizes a B cell or T cell surface.

2. The conjugate of claim 1, wherein A is a peptide represented by any one of the amino acid sequences of SEQ ID NO: 1 to SEQ ID NO: 15.

3. The conjugate of claim 1, wherein D is a peptide selected from the group consisting of 4 to 12 arginines, 4 to 12 lysines, and 2 cysteines, or a combination thereof.

4. The conjugate of claim 1, wherein E is a peptide represented by the amino acid sequence of SEQ ID NO: 17.

5. The conjugate of claim 1, further comprising C, a peptide that facilitates endosomal escape, between B and D.

6. The conjugate of claim 5, wherein C is a peptide consisting of 4 to 12 histidines.

7. The conjugate of claim 5, wherein the A-B-D-E or the A-B-C-D-E is produced by a chemical synthesis technique or a recombinant expression technique.

8. A nanoparticle in which mRNA is bound to the peptide-based conjugate of any one of claims 1 to 7.

9. The nanoparticle of claim 8, wherein the mRNA encodes a target protein, recombinant protein, or viral antigen whose expression is to be increased.

10. The nanoparticle of claim 8, wherein the mRNA is bound to A in Structural Formula 1, and then the nanoparticle is formed by self-assembly of the peptide-based conjugate.

11. The nanoparticle of claim 10, which has a size of 10 to 200 nm.

12. The nanoparticle of claim 8, wherein a molecular weight ratio between the mRNA and the conjugate is 1:1 to 1:100.

13. A composition for delivering mRNA into B cells or T cells comprising the nanoparticle of claim 8.

14. A pharmaceutical composition for preventing or treating a viral infection comprising the nanoparticle of claim 8.

15. The pharmaceutical composition of claim 14, wherein the viral infection is a cold, flu (influenza), infectious mononucleosis, cytomegalovirus infection, measles, polio, yellow fever, dengue fever, hepatitis B, hepatitis C, AIDS, or COVID-19.

FIG. 1

RNA-binding peptide | Amphipathic polypeptide or polymer | OR | Cell-penetrating peptide | Immune cell-recognizing peptide

FIG. 2

FIG. 3

FIG. 3 (continued)

**B**

FIG. 3 (continued)

# C

Intensity Distribution
## N/P ratio 1:1

Cumulants Results

Diameter          (d)    : 329.7          (nm)
Polydispersity Index (P.I.) : 0.041

Intensity Distribution
## N/P ratio 5:1

Cumulants Results

Diameter          (d)    : 175.7          (nm)
Polydispersity Index (P.I.) : 0.106

Intensity Distribution
## N/P ratio 10:1

Cumulants Results

Diameter          (d)    : 120.9          (nm)
Polydispersity Index (P.I.) : 0.317

Intensity Distribution
## N/P ratio 20:1

Cumulants Results

Diameter          (d)    : 79.5          (nm)
Polydispersity Index (P.I.) : 0.245

FIG. 4

FIG. 5

FIG. 6

FIG. 7

FIG. 8

FIG. 9

FIG. 10

FIG. 11

## INTERNATIONAL SEARCH REPORT

| | International application No. |
|---|---|
| | **PCT/KR2023/020203** |

| A. | CLASSIFICATION OF SUBJECT MATTER |
|---|---|

**A61K 48/00**(2006.01)i; **A61K 47/69**(2017.01)i; **A61K 9/51**(2006.01)i; **A61K 31/7088**(2006.01)i; **A61P 31/12**(2006.01)i

According to International Patent Classification (IPC) or to both national classification and IPC

| B. | FIELDS SEARCHED |
|---|---|

Minimum documentation searched (classification system followed by classification symbols)

A61K 48/00(2006.01); A61K 38/00(2006.01); A61K 39/215(2006.01); A61K 39/35(2006.01); A61K 47/42(2006.01); A61K 47/48(2006.01); B82B 1/00(2006.01); B82Y 5/00(2011.01); C07K 14/00(2006.01); C07K 19/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 펩타이드(peptide), 지질 나노입자(lipid nanoparticle, LNP), 세포막 투과성(cell membrane permeability), T 세포(T cell), B 세포(B cell), 양친매성(amphipathy), 엔도솜(endosome)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | KR 10-2015-0039259 A (KOREA INSTITUTE OF SCIENCE AND TECHNOLOGY) 10 April 2015 (2015-04-10)<br>See abstract; claim 1; and paragraphs [0004], [0008], [0014], [0019], [0040] and [0046]-[0048]. | 1-15 |
| A | WO 2021-188969 A2 (BIONTECH US INC.) 23 September 2021 (2021-09-23)<br>See abstract; claims 1, 28-30, 47-49, 68-69 and 76-80; and paragraphs [0007]-[0009]. | 1-15 |
| A | KR 10-2012-0102586 A (CUREVAC GMBH) 18 September 2012 (2012-09-18)<br>See entire document. | 1-15 |
| A | KR 10-0951719 B1 (SEOUL NATIONAL UNIVERSITY INDUSTRY FOUNDATION) 07 April 2010 (2010-04-07)<br>See entire document. | 1-15 |
| A | KR 10-2022-0139079 A (STEM MEDITECH CO., LTD.) 14 October 2022 (2022-10-14)<br>See entire document. | 1-15 |

| ☐ Further documents are listed in the continuation of Box C. | ☑ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **19 March 2024** | **20 March 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

| **INTERNATIONAL SEARCH REPORT** | International application No. |
| --- | --- |
| | **PCT/KR2023/020203** |

| **Box No. I** | **Nucleotide and/or amino acid sequence(s) (Continuation of item 1.c of the first sheet)** |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/KR2023/020203**

| Patent document cited in search report | | | Publication date (day/month/year) | Patent family member(s) | | | Publication date (day/month/year) |
|---|---|---|---|---|---|---|---|
| KR | 10-2015-0039259 | A | 10 April 2015 | KR | 10-1559974 | B1 | 15 October 2015 |
| WO | 2021-188969 | A2 | 23 September 2021 | EP | 4121104 | A2 | 25 January 2023 |
| | | | | JP | 2023-518821 | A | 08 May 2023 |
| | | | | KR | 10-2023-0004508 | A | 06 January 2023 |
| | | | | US | 2023-0083931 | A1 | 16 March 2023 |
| | | | | US | 2023-0141371 | A1 | 11 May 2023 |
| | | | | WO | 2021-188969 | A3 | 25 November 2021 |
| KR | 10-2012-0102586 | A | 18 September 2012 | CN | 102573919 | A | 11 July 2012 |
| | | | | CN | 102573919 | B | 06 August 2014 |
| | | | | EP | 2810661 | A2 | 10 December 2014 |
| | | | | JP | 2013-515674 | A | 09 May 2013 |
| | | | | JP | 5813642 | B2 | 17 November 2015 |
| | | | | KR | 10-1572249 | B1 | 26 November 2015 |
| | | | | KR | 10-1790236 | B1 | 26 October 2017 |
| | | | | KR | 10-2014-0090689 | A | 17 July 2014 |
| | | | | KR | 10-2015-0067401 | A | 17 June 2015 |
| | | | | US | 10751424 | B2 | 25 August 2020 |
| | | | | US | 2011-0053829 | A1 | 03 March 2011 |
| | | | | US | 2012-0219573 | A1 | 30 August 2012 |
| | | | | US | 2014-0294877 | A1 | 02 October 2014 |
| | | | | US | 2016-0317679 | A1 | 03 November 2016 |
| | | | | US | 2018-0161457 | A1 | 14 June 2018 |
| | | | | US | 2020-0338215 | A1 | 29 October 2020 |
| | | | | US | 8703906 | B2 | 22 April 2014 |
| | | | | US | 9314535 | B2 | 19 April 2016 |
| | | | | US | 9907862 | B2 | 06 March 2018 |
| | | | | WO | 2011-026641 | A1 | 10 March 2011 |
| KR | 10-0951719 | B1 | 07 April 2010 | EP | 2197783 | A2 | 23 June 2010 |
| | | | | EP | 2197783 | B1 | 11 July 2018 |
| | | | | KR | 10-2009-0034240 | A | 07 April 2009 |
| | | | | US | 2010-0298536 | A1 | 25 November 2010 |
| | | | | WO | 2009-045063 | A2 | 09 April 2009 |
| KR | 10-2022-0139079 | A | 14 October 2022 | CN | 117157308 | A | 01 December 2023 |
| | | | | EP | 4321527 | A1 | 14 February 2024 |
| | | | | WO | 2022-215946 | A1 | 13 October 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20060083780 A
- US 20060240554 A
- US 20080020058 A
- US 20090263407 A
- US 20090285881 A
- WO 2009086558 A
- WO 2009127060 A
- WO 2009132131 A
- WO 2010042877 A
- WO 2010054384 A
- WO 2010054401 A
- WO 2010054405 A
- WO 2010054406 A
- WO 2010105209 A
- US 20200207834 A1
- KR 100951719

### Non-patent literature cited in the description

- Remington's Pharmaceutical Science. Mack Publishing Company